# EUROPEAN PATENT APPLICATION

(11) **EP 4 487 862 A1**
(43) Date of publication of application: **08.01.2025**
(21) Application number: 23763692.3
(22) Date of filing: 28.02.2023
(51) Int. Cl.: A61K 36/81, A61K 36/287, A61P 3/04, A23L 33/105

(54) **COMPOSITION FOR PREVENTING, ALLEVIATING OR TREATING OBESITY, COMPRISING, AS ACTIVE INGREDIENT, COMPLEX (ASC COMPLEX) OF WITHANIA SOMNIFERA EXTRACT AND SIBERIAN CHRYSANTHEMUM EXTRACT**

(30) Priority: 02.03.2022 KR 20220026947
(71) Applicant: HLscience Co., Ltd, Hwaseong-si, Gyeonggi-do 18329 (KR)
(72) Inventor: LEE, Hae-Yeon, Uiwang-si, Gyeonggi-do 16000 (KR); KIM, Jong-Lae, Uiwang-si, Gyeonggi-do 16009 (KR); IM, Sung Bin, Anyang-si, Gyeonggi-do 14038 (KR); KIM, So Young, Seongnam-si, Gyeonggi-do 13262 (KR); PARK, Mi-Ryeong, Uiwang-si, Gyeonggi-do 16077 (KR)
(74) Representative: Graf von Stosch Patentanwaltsgesellschaft mbH
(86) International application number: PCT/KR2023/002819
(87) International publication number: WO 2023/167493

(57) **Abstract**

The present invention relates to a composition for preventing, improving or treating obesity, comprising a complex of an extract of Ashwagandha *(Withania somnifera)* and an extract of Siberian chrysanthemum (*Chrysanthemum zawadskii Herbich var. latilobum (Maxim.) Kitamura*) as an active ingredient.

The complex provided from the present invention has very excellent effects of inhibiting differentiation of adipocytes, inhibiting lipid accumulation in adipocytes, and inhibiting pancreatic lipase, and thus it can be used in a pharmaceutical composition for preventing or treating obesity and a health functional food and food composition for preventing or improving obesity.

## Description

### [TECHNICAL FIELD]

This application claims priority based on Korean Application No. 10-2022-0026947 filed on March 02, 2022, and all contents disclosed in the specification and drawings of the application are incorporated into this application.

The present invention relates to a composition for preventing, improving or treating obesity comprising natural extracts as an active ingredient.

### [BACKGROUND ART]

Obesity refers to a medical condition in which excess lipid is accumulated due to an imbalance in energy metabolism. It can be defined as a state of calorie imbalance in which excess energy is accumulated in adipose tissue in the form of neutral fat because caloric intake exceeds the energy required for physical activity and growth. It is known that many factors such as genetics, lack of exercise, excessive calorie intake, intestinal microbial imbalance, and poor quality of sleep work in combination to cause obesity.

To date, many methods for treating obesity have been attempted, and in Korea, according to the obesity treatment guidelines of the Korean Society of Obesity (2018), patients with a BMI of 25 kg/m² or more are subject to drug therapy, and patients with a BMI of 35 kg/m² or more, or with a BMI of 30 kg/m² or more and an obesity comorbidity are recommended to consider surgery. Anti-obesity drugs approved by the US FDA are orlistat, locarserin, naltrexone/bupropion, phentermine/topiramate, liraglutide, etc. However, in the case of drug therapy, it has been reported that side effects such as depression, anxiety, headache, dizziness, nausea, and fatigue occur, and issues regarding safety and effectiveness are constantly being raised. In addition, in the case of surgical treatment, there is a limitation that exercise and diet must be combined for a long time after surgery or procedure, so research on the anti-obesity effect and safety of various natural substances is being actively conducted domestically and internationally.

Therefore, it is necessary to develop natural substances for improving or treating obesity that is safe for the human body and has excellent anti-obesity effects.

### [DISCLOSURE]

### [TECHNICAL PROBLEM]

The present inventors have completed the present invention upon finding that the combination of Ashwagandha extract and Siberian chrysanthemum extract can exhibit excellent effects of inhibiting adipocyte differentiation, inhibiting lipid/fat accumulation in adipocytes, and inhibiting pancreatic lipase activity, thereby providing an anti-obesity effect, and can exhibit an effect of preventing, improving and treating obesity.

Accordingly, an object of the present invention is to provide a pharmaceutical composition for preventing or treating obesity, comprising a complex of Ashwagandha extract and Siberian chrysanthemum extract as an active ingredient.

Another object of the present invention is to provide a health functional food and food composition for preventing or improving obesity comprising a complex of Ashwagandha extract and Siberian chrysanthemum extract as an active ingredient.

Another object of the present invention is to provide pharmaceutical products, health functional food products and food products comprising the composition.

More specifically, an object of the present invention is to provide the following embodiments.
Embodiment 1. A pharmaceutical composition for preventing or treating obesity, comprising a complex of an extract of Ashwagandha (*Withania somnifera*) and an extract of Siberian chrysanthemum *(Chrysanthemum zawadskii Herbich var. latilobum (Maxim.) Kitamura*) as an active ingredient; a method of preventing or treating obesity, comprising administering a complex of an extract of Ashwagandha (*Withania somnifera*) and an extract of Siberian chrysanthemum (*Chrysanthemum zawadskii Herbich var. latilobum (Maxim.) Kitamura*) to a subject in need thereof; or use of a complex of an extract of Ashwagandha (*Withania somnifera*) and an extract of Siberian chrysanthemum (*Chrysanthemum zawadskii Herbich var. latilobum (Maxim.) Kitamura*) in the manufacture of a pharmaceutical product for preventing or treating obesity.
Embodiment 2. The pharmaceutical composition, method, or use according to Embodiment 1, wherein the extract of Ashwagandha and the extract of Siberian chrysanthemum are extracted with a solvent selected from the group consisting of water, lower alcohols having 1 to 4 carbon atoms, and mixed solvents thereof.
Embodiment 3. The pharmaceutical composition, method, or use according to any of the preceding embodiments, wherein the extract of Ashwagandha and the extract of Siberian chrysanthemum are extracted at 40 to 100 °C.
Embodiment 4. The pharmaceutical composition, method, or use according to any of the preceding embodiments, wherein the complex of the extract of Ashwagandha and the extract of Siberian chrysanthemum is a dry powder.
Embodiment 5. The pharmaceutical composition, method, or use according to any of the preceding embodiments, wherein the extract of Ashwagandha and the extract of Siberian chrysanthemum are mixed in the complex in a weight ratio of 1:1 to 5:1 (extract of Ashwagandha : extract of Siberian chrysanthemum).
Embodiment 6. The pharmaceutical composition, method, or use according to any of the preceding embodiments, wherein the complex of the extract of Ashwagandha and the extract of Siberian chrysanthemum has an inhibitory effect on differentiation of adipocytes, an inhibitory effect on lipid accumulation in adipocytes, and an inhibitory activity on pancreatic lipase.
Embodiment 7. A pharmaceutical product comprising the composition according to any of the preceding embodiments.
Embodiment 8. A food composition for preventing or improving obesity, comprising a complex of an extract of Ashwagandha (*Withania somnifera)* and an extract of Siberian chrysanthemum (*Chrysanthemum zawadskii Herbich var. latilobum (Maxim.) Kitamura*) as an active ingredient; or use of a complex of an extract of Ashwagandha *(Withania somnifera*) and an extract of Siberian chrysanthemum (*Chrysanthemum zawadskii Herbich var. latilobum (Maxim.) Kitamura*) in the manufacture of a food product for preventing or improving obesity.
Embodiment 9. The food composition or use according to Embodiment 8, wherein the extract of Ashwagandha and the extract of Siberian chrysanthemum are extracted with a solvent selected from the group consisting of water, lower alcohols having 1 to 4 carbon atoms, and mixed solvents thereof.
Embodiment 10. The food composition or use according to any of Embodiments 8 to 9, wherein the extract of Ashwagandha and the extract of Siberian chrysanthemum are extracted at 40 to 100 °C.
Embodiment 11. The food composition or use according to any of Embodiments 8 to 10, wherein the complex of the extract of Ashwagandha and the extract of Siberian chrysanthemum is a dry powder.
Embodiment 12. The food composition or use according to any of Embodiments 8 to 11, wherein the extract of Ashwagandha and the extract of Siberian chrysanthemum are mixed in the complex in a weight ratio of 1:1 to 5:1 (extract of Ashwagandha : extract of Siberian chrysanthemum).
Embodiment 13. The food composition or use according to any of Embodiments 8 to 12, wherein the complex of the extract of Ashwagandha and the extract of Siberian chrysanthemum has an inhibitory effect on differentiation of adipocytes, an inhibitory effect on lipid accumulation in adipocytes, and an inhibitory activity on pancreatic lipase.
Embodiment 14. A health functional food product comprising the composition according to any of Embodiments 8 to 13.
Embodiment 15. A food product comprising the composition according to any of Embodiments 8 to 13.

Further objects and advantages of the present invention will become more apparent from the following detailed description of the invention, claims and drawings.

### [TECHNICAL SOLUTION]

In order to achieve the above object, the present invention provides a composition for preventing, improving or treating obesity, comprising a complex of an extract of Ashwagandha (*Withania somnifera*) and an extract of Siberian chrysanthemum (*Chrysanthemum zawadskii Herbich var. latilobum (Maxim.) Kitamura*) as an active ingredient.

In the present invention, the Ashwagandha extract and the Siberian chrysanthemum extract may be characterized in that they are extracted with a solvent selected from the group consisting of water, an organic solvent, or a mixed solvent thereof.

In the present invention, the Ashwagandha extract and the Siberian chrysanthemum extract may be characterized in that they are extracted at a low-temperature of 40 to 100 °C, more preferably 40 to 80 °C.

In the present invention, the complex of Ashwagandha extract and Siberian chrysanthemum extract may be characterized in that it is a dry powder.

In the present invention, the Ashwagandha extract and the Siberian chrysanthemum extract may be characterized in that they are mixed in the complex in a weight ratio of 1:1 to 5:1.

In the present invention, the complex of the Ashwagandha extract and the Siberian chrysanthemum extract may be characterized by having an effect of inhibiting differentiation of adipocytes, an effect of inhibiting fat accumulation in adipocytes, and an inhibitory activity of pancreatic lipase.

In addition, the present invention provides a pharmaceutical product, a health functional food product and a food product comprising the composition.

### [EFFECTS]

The complex provided from the present invention has excellent effects of inhibiting differentiation of adipocytes, inhibiting fat accumulation in adipocytes, and inhibiting pancreatic lipase, and thus it can be used in a pharmaceutical composition for preventing or treating obesity and a health functional food product and a food composition for preventing or improving obesity.

### [BRIEF DESCRIPTION OF THE DRAWINGS]

Figure 1 is a photomicrograph showing the effect of inhibiting differentiation of adipocytes by treating 3T3-L1 cells with a complex of Ashwagandha extract and Siberian chrysanthemum extract.
Figure 2 is a graph showing the effect of inhibiting fat accumulation in adipocytes by treating 3T3-L1 cells with a complex of Ashwagandha extract and Siberian chrysanthemum extract.
Figure 3 is a graph showing the effect of Example 2 on inhibiting fat accumulation in adipocytes.

### [MODE FOR INVENTION]

Unless defined otherwise, all technical and scientific terms used herein have the same meaning as commonly understood by one of ordinary skill in the art to which this invention belongs. In general, the nomenclatures used herein are those well known and commonly used in the art.

Throughout the present specification, when a certain component is said to "comprise", it means that it may further comprise other components without excluding other components unless otherwise stated.

According to one embodiment of the present invention, the present invention relates to a composition for preventing, improving or treating obesity, comprising a complex of an extract of Ashwagandha (*Withania somnifera*) and an extract of Siberian chrysanthemum (*Chrysanthemum zawadskii Herbich var. latilobum (Maxim.) Kitamura*) as an active ingredient.

The extracts according to the present invention may be obtained through various extraction methods known in the field to which the present invention pertains, and specifically may be extracted with a solvent selected from the group consisting of water, an organic solvent, or a mixed solvent thereof.

The water may be alkaline water, and the organic solvent may be an anhydrous or hydrous lower alcohol having 1 to 4 carbon atoms, for example, a polar organic solvent such as methanol, ethanol, propanol, n-butanol, or acetone; non-polar organic solvents such as ether, hexane, benzene, chloroform, and ethyl acetate; vegetable organic solvents such as soybean oil and sesame oil; and mixtures thereof. The organic solvent may be used alone or in combination of two or more.

As the solvent used for the extraction, one or more organic solvents selected from among methanol, ethanol, propanol, n-butanol, and acetone, or a mixed solvent including water in these solvents may be used. Among them, extraction with a mixed solvent of water and ethanol is more preferable in terms of stably extracting the active ingredient. The concentration of ethanol in the mixed solvent of water and ethanol may be 10 to 90%, preferably 30 to 90%, and more preferably 50 to 80%.

Specifically, the content of the extraction solvent may be 1 to 300 times, more preferably 5 to 150 times, and most preferably 10 to 50 times the solid weight of the extraction material.

In addition, the extract may be extracted by heating or at room temperature under conditions in which the active ingredients of the extract are not destroyed or destruction is minimized. The extraction method is not particularly limited, and depending on the type of extract and the extraction method, for example, low-temperature extraction, bath water extraction, reflux cooling extraction, ultrasonic extraction, and cold extraction may be used.

The number of extractions in the extraction step may be specifically repeated 1 to 5 times. In this case, the extraction efficiency of the active ingredient can be further improved.

For example, in the step of extracting, after adding the extraction solvent to the extraction material, extraction may be performed at a low temperature of 40 to 100 °C, more preferably 40 to 80 °C, for 2 to 80 hours. At this time, the low-temperature extraction method is a method of extracting at low temperature by applying heat. When using the low-temperature extraction method, it is preferable to minimize the destruction of active ingredients by heat.

In another method, the extraction may be performed by adding an extraction solvent to the extraction material and then performing extraction at 80 to 100 °C for 0.5 to 72 hours in hot water.

In another method, the extraction may be performed by heating under reflux for 4 to 20 hours at 50 to 100 °C after adding the extraction material and the extraction solvent to an extractor equipped with a reflux condenser. At this time, the heating and reflux cooling extraction method is a method of extracting by heating and refluxing and then cooling. Specifically, since solvent loss may occur due to vaporization due to heating, the cooling process is performed together to minimize solvent loss.

In another method, the extraction may be performed by adding an extraction solvent to the extraction material, and then immersing at 5 to 37 °C for 1 to 15 days and cooling.

In another method, the extraction step may be performed by adding 10 to 50 times the weight of ethanol to the extracted material based on the dry solids of the extracted material, extracting at 20 to 80 °C for 1 to 72 hours, filtering and concentrating to get an extract in a concentrated state.

In addition, the extraction material of the present invention includes not only the extraction material extracted by the above-described extraction solvent, but also the extraction material extracted through a conventional purification process. For example, active fractions obtained through various additional purification methods such as a fraction obtained by passing through an ultrafiltration membrane having a certain molecular weight cut-off value, or by various chromatography (designed for separation according to size, charge, hydrophobicity or hydrophilicity), etc. are also included in the extract of the present invention.

Filtration is a process of removing floating solid particles from the extract, and particles may be filtered out using cotton, nylon, paper, etc., or ultrafiltration, cryofiltration, centrifugation, etc. may be used, but is not limited thereto.

The step of concentrating and then drying the filtrate includes, but is not limited to, freeze drying, vacuum drying, hot air drying, spray drying, reduced pressure drying, foam drying, high frequency drying, infrared drying, and the like. In some cases, a step of pulverizing the finally dried extract may be added.

According to one embodiment of the present invention, the extract may be processed and used in various forms, such as an extracted solution, a powder obtained by drying the extract, and a concentrate obtained by filtering and concentrating the extract. In addition, the extract is not particularly limited, but may be used, for example, in the form of a tincture containing hydrous alcohol as a leaching solvent, a concentrate, an extractives, a fluid extract, etc.

According to another embodiment of the present invention, the Ashwagandha extract prepared as described above may contain 3 to 9 mg/g of Withanoside IV. In addition, the Siberian chrysanthemum extract prepared as above may contain 15 to 50 mg/g of Linarin.

According to another embodiment of the present invention, the complex of the Ashwagandha extract and the Siberian chrysanthemum extract may be prepared by mixing the Ashwagandha extract and the Siberian chrysanthemum extract in a preferable weight ratio of 1:1 to 5:1 (Asiaganda extract: Siberian chrysanthemum extract), and it may be most preferable to carry out the mixing in a weight ratio of 3:1 to 5:1 (Asiaganda extract: Siberian chrysanthemum extract) in terms of improving all of the effects of inhibiting differentiation of adipocytes, inhibiting lipid accumulation in adipocytes, and inhibiting pancreatic lipase. If it is out of the above range, it is not desirable because the effects of inhibiting differentiation of adipocytes, inhibiting lipid accumulation in adipocytes, and inhibiting pancreatic lipase are reduced, and thus the function as a composition for preventing, improving or treating obesity is reduced.

Since the complex of the Ashwagandha extract and the Siberian chrysanthemum extract can impart all the effects of inhibiting differentiation of adipocytes, inhibiting lipid accumulation in adipocytes, and inhibiting pancreatic lipolytic enzyme, it can not only suppress lipid accumulation, but also inhibit lipid absorption in the body. By inhibiting it, the anti-obesity effect can be implemented.

As described above, the Ashwagandha extract and the Siberian chrysanthemum extract are excellent in inhibiting differentiation of adipocytes, inhibiting lipid accumulation in adipocytes, and inhibiting pancreatic lipolytic enzyme, and thus can be used in a pharmaceutical composition for preventing or treating obesity and in a health functional food product and a food composition for preventing or improving obesity.

According to one embodiment of the present invention, the pharmaceutical composition may further include suitable carriers, excipients, and diluents commonly used in the preparation of pharmaceutical compositions, as well as the Ashwagandha extract and the Siberian chrysanthemum extract as active ingredients. In addition, solid or liquid formulation additives may be used for the preparation of pharmaceutical compositions, but are not limited thereto.

As the excipient, lactose, sucrose, white sugar, glucose, oligosaccharide, corn starch, starch, talc, sorbitol, crystalline cellulose, dextrin, calcium carbonate, silicon dioxide, and the like may be used. Examples of the binder include polyvinyl alcohol, polyvinyl ether, methyl cellulose, ethyl cellulose, gum arabic, gelatin, hydroxypropyl cellulose, hydroxypropyl methyl cellulose, calcium citrate, dextrin, and pectin. Magnesium stearate, talc, polyethylene glycol, silica and hydrogenated vegetable oil may be used as the lubricant. As the coloring agent, any colorant can be used as long as it is permitted to be added to ordinary pharmaceuticals. These tablets and granules may be appropriately coated such as sugar coating and gelatin coating according to other needs. In addition, preservatives and antioxidants may be added as needed.

In addition, the pharmaceutical composition of the present invention can be prepared in any formulation commonly prepared in the art, and the form of the formulation is not particularly limited, but powders, granules, tablets, capsules, suspensions, emulsions, oral formulations such as syrups and aerosols, external preparations, suppositories and sterile injection solutions may be formulated and used according to conventional methods, but are not limited thereto.

The preferred dosage of the pharmaceutical composition of the present invention may vary depending on the condition and weight of the patient, the severity of the disease, the type of wound, the application site, number of administrations, the administration time, the type of dosage form and supplements, and the like. The dosage is not particularly limited, but usually the pharmaceutical composition of the present invention may be 0.0001 to 500 mg per day. Administration may be administered once a day, divided into 2 to 3 times a day at appropriate intervals, or may be administered intermittently at intervals of several days.

In addition, the dosage of the pharmaceutical composition of the present invention depends on the route of administration, the patient's age, sex, weight, patient's severity, wound type, application site, number of administrations, administration time, dosage form, patient's condition and type of adjuvant, etc. It can be appropriately adjusted according to various related factors of, and can be usefully used as a pharmaceutical composition for the prevention or treatment of obesity.

The composition obtained from the method described above exhibits effects of inhibiting differentiation of adipocytes, inhibiting lipid accumulation in adipocytes, and inhibiting pancreatic lipolytic enzyme, and thus can be used in providing a pharmaceutical product excellent in preventing and treating obesity.

According to one embodiment of the present invention, the health functional food and food composition can be used as food, food additives, beverages, beverage additives, fermented milk, health functional food, and the like. When used as food, food additives, beverages, beverage additives, or health functional foods, it may be various foods, fermented milk, meat, beverages, chocolate, snacks, confectionery, pizza, ramen, other noodles, chewing gum, ice cream, alcoholic beverages, vitamin complex, alcoholic products and other health functional foods, but is not limited thereto.

When the complex of Ashwagandha extract and Siberian chrysanthemum extract of the present invention is prepared as a health functional food and food composition, it may contain not only the Ashwagandha extract and the Siberian chrysanthemum extract, but also components commonly added during food preparation as active ingredients.

The additional ingredients include, for example, proteins, carbohydrates, fats, nutrients, seasonings and flavors. The carbohydrates include common sugars such as monosaccharides (e.g. glucose, fructose, etc.), disaccharides (e.g. maltose, sucrose, oligosaccharides, etc.) and polysaccharides (e.g. dextrin, cyclodextrin, etc.), xylitol, sorbitol and sugar alcohols such as erythritol. As flavoring agents, natural flavoring agents (thaumatin, stevia extract (e.g. rebaudioside A, glycyrrhizin, etc.)) and synthetic flavoring agents (saccharin, aspartame, etc.) may be used.

For example, when the health functional food and food composition of the present invention are prepared as a drink, not only Ashwagandha extract and Siberian chrysanthemum extract, but also pectin, citric acid, high fructose corn syrup, sugar, glucose, acetic acid, malic acid, fruit juice, etc. can be included.

The health functional food and food composition of the present invention may include processed forms of all natural materials such as food, functional food, nutritional supplement, health food and food additives, etc. Health functional foods and food compositions of the above types may be prepared in various forms according to conventional methods known in the art.

For example, as a health food, the complex of Ashwagandha extract and Siberian chrysanthemum extract may be prepared and consumed in a form of tea, juice, and drink, or may be consumed in a granulated, encapsulated, or powdered form. In addition, the natural extracts of the present invention can be added in preparing foods such as beverages (including alcoholic beverages), fruits and their processed foods (for example, canned fruit, bottled fruit, jam, marmalade, etc.), fish, meat and their processed foods (for example, ham, sausage, corned beef, etc.), breads and noodles (e.g. udon, buckwheat noodles, ramen, spaghetti, macaroni, etc.), fruit juice, various drinks, cookies, taffy, dairy products (e.g. yogurt, fermented milk, butter, cheese, etc.), edible vegetable oils, margarine, vegetable protein, retort food, frozen food, various seasonings (e.g. soybean paste, soy sauce, sauce, etc.), etc. In addition, in order to use the complex of Ashwagandha extract and Siberian chrysanthemum extract of the present invention as a food additive, it can be prepared and used in a form of powder or concentrate.

The amount of use of the functional food and food composition for preventing or improving obesity of the present invention can be appropriately adjusted according to individual differences such as age, health status, formulations, and forms, and can be used as a health functional food and food composition for preventing or improving obesity.

The composition obtained from the method described above exhibits effects of inhibiting differentiation of adipocytes, inhibiting lipid accumulation in adipocytes, and inhibiting pancreatic lipolytic enzyme, thereby providing excellent health functional food products and food products for preventing or improving obesity.

Hereinafter, the present invention will be described in more detail through examples. These examples are only for explaining the present invention in more detail, and it will be apparent to those of ordinary skill in the art that the scope of the present invention is not limited by these examples according to the gist of the present invention.

### <Examples 1 to 3> Preparation of a complex of Ashwagandha extract and Siberian chrysanthemum extract

After pulverizing 20 g of dried Ashwagandha roots with a grinder, 80% ethanol was added in an amount 15 times the weight of the Ashwagandha and extracted twice at 60 °C for 8 hours by a low-temperature extraction method. The obtained extracts were mixed, respectively, and the filtrate obtained by filtering with a 400 mesh filter was concentrated to 60 brix in a rotary vacuum concentrator and freeze-dried to prepare an Ashwagandha extract in a powder form. In addition, 50% ethanol was added to 20 g of dried Siberian chrysanthemum (*Chrysanthemum zawadskii Herbich var. latilobum (Maxim.) Kitamura*, whole plant) in an amount of 10 times the weight of the dried whole plant of Siberian chrysanthemum, and extracted twice at 80 °C for 4 hours by a low temperature extraction method. The obtained extracts were mixed, respectively, and the filtrate obtained by filtering with a 400 mesh filter was concentrated to 60 brix in a rotary vacuum concentrator and freeze-dried, and thereby a Siberian chrysanthemum extract was prepared in a powder form. The powdered Ashwagandha extract and Siberian chrysanthemum extract prepared as described above were combined in a weight ratio as shown in Table 1 below to prepare a complex of Ashwagandha extract and Siberian chrysanthemum extract as a final product.

**[Table 1]**

| Classification | Complex Ratio of Ashwagandha extract: Siberian chrysanthemum extract | Ashwagandha extract (% by weight) | Siberian chrysanthemum extract (% by weight) |
|---|---|---|---|
| Example 1 | 1:1 | 50 | 50 |
| Example 2 | 3:1 | 75 | 25 |
| Example 3 | 5:1 | 83.33 | 16.67 |

### <Comparative Examples 1 to 6> Preparation of a complex of Ashwagandha extract and Siberian chrysanthemum extract

Each of the extracts was prepared in the same manner as in the above example, and mixed in a weight ratio as shown in Table 2 below to prepare a complex of Ashwagandha extract and Siberian chrysanthemum extract as a final product.

**[Table 2]**

| Classification | Complex Ratio of Ashwagandha extract: Siberian chrysanthemum extract | Ashwagandha extract (% by weight) | Siberian chrysanthemum extract (% by weight) |
|---|---|---|---|
| Comparative Example 1 | 7:1 | 87.5 | 12.5 |
| Comparative Example 2 | 1:7 | 12.5 | 87.5 |
| Comparative Example 3 | 1:5 | 16.67 | 83.33 |
| Comparative Example 4 | 1:3 | 25 | 75 |
| Comparative Example 5 | 1:0 | 100 | 0 |
| Comparative Example 6 | 0:1 | 0 | 100 |

### <Experimental Example 1> Analyzation of components of Ashwagandha extract and Siberian chrysanthemum extract

In order to analyze the main component content of the Ashwagandha extract and the Siberian chrysanthemum extract and standardize the extracts, three samples were prepared for each Ashwagandha extract (Comparative Example 5-1, Comparative Example 5-2, Comparative Example 5-3) and Siberian chrysanthemum extract (Comparative Example 6-1, Comparative Example 6-2, Comparative Example 6-3).

Components of the Ashwagandha extract were analyzed using a high-performance liquid chromatography method, and the main component was set as Withanoside IV. Capcellpak C18 MG (4.6 mm × 250 mm, 5 µm) was used as a column, and analysis was performed by a gradient method using 1 mM potassium phosphate solution (A) and acetonitrile (B) as a mobile phase. Withanoside IV was detected at a wavelength of 227 nm with a UV detector. As a result, as shown in Table 3 below, the content of Withanoside IV in the Ashwagandha extract prepared according to Comparative Example 5 was found to be in the range of 3 to 9 mg/g.

**[Table 3]**

| Classification | Content of Withanoside IV (mg/g) |
|---|---|
| Comparative Example 5-1 | 3.07 |
| Comparative Example 5-2 | 5.25 |
| Comparative Example 5-3 | 8.97 |

Components of the Siberian chrysanthemum extract were analyzed using a high-performance liquid chromatography method, and the main component was set as Linarin. It was analyzed by the gradient method using Zorbax Eclipse plus C18 (4.6 mm × 250 mm, 5 µm) as a column, and purified water containing 0.1% formic acid (A) and acetonitrile containing 0.1% formic acid (B) as mobile phases. Linarin was detected at a wavelength of 310 nm with a UV detector. As a result, as shown in Table 4 below, the content of Linarin in the Siberian chrysanthemum extract prepared according to Comparative Example 6 was found to be in the range of 15 to 50 mg/g.

**[Table 4]**

| Classification | Content of Linarin (mg/g) |
|---|---|
| Comparative Example 6-1 | 14.98 |
| Comparative Example 6-2 | 25.85 |
| Comparative Example 6-3 | 50.39 |

### <Experimental Example 2> Cytotoxicity test: WST (Water-soluble tetrazolium salt) assay

A cytotoxicity test was conducted using 3T3-L1 cells, a mouse-derived pre-adipocyte cell line, to determine whether they exhibit toxicity to pre-adipocytes using Examples 1 to 3 and Comparative Examples 1 to 6.

In order to determine the toxicity to 3T3-L1 cells, a mouse-derived preadipocyte cell line, the cells were dispensed at a concentration of 5×10⁴ cells/well in a 24-well plate using DMEM (Dulbecco's Modified Eagle Medium) medium, and then incubated for 24 hours under the condition of 37°C and 5% CO₂. For the cell cultured for 24 hours, Examples 1 to 3 and Comparative Examples 1 to 6 were mixed with the culture medium by concentration, treated in each well, cultured for 24 hours under the condition of 37°C and 5% CO₂, and then the culture medium was removed. After treating each well with WST-1 assay solution and reacting in an incubator for 2 hours, the absorbance was measured at 450 nm with a micro-plate reader, and the cell viability was calculated as in Equation 1 below. Cell viability (%) = [(absorbance of sample treated group / absorbance of untreated group) × 100]

**[Table 5]**

| Sample (complex ratio) | Treatment concentration | Cell viability (%) |
|---|---|---|
| Example 1 (1:1) | 60 *µ*g/mL | 100.40±3.21 |
| Example 2 (3:1) | 60 *µ*g/mL | 100.16±2.07 |
| Example 3 (5:1) | 60 *µ*g/mL | 97.64±1.21 |
| Comparative Example 1 (7:1) | 60 *µ*g/mL | 98.45±1.70 |
| Comparative Example 2 (1:7) | 60 *µ*g/mL | 107.19±2.73 |
| Comparative Example 3 (1:5) | 60 *µ*g/mL | 102.87±1.37 |
| Comparative Example 4 (1:3) | 60 *µ*g/mL | 100.52±2.66 |
| Comparative Example 5 (1:0) | 60 *µ*g/mL | 95.20±1.18 |
| Comparative Example 6 (0:1) | 60 *µ*g/mL | 106.35±2.16 |

As a result, as shown in Table 5, it was found that cytotoxicity was not observed at the treatment concentration of 60 µg/mL of each Examples 1 to 3 and Comparative Examples 1 to 6, so that it could be used as a safe material for pharmaceuticals and foods.

### <Experimental Example 3> Inhibition of lipid accumulation: Inhibition of differentiation of adipocytes and inhibition of lipid accumulation in adipocytes

In order to examine the lipid accumulation inhibitory effect at the cellular level using Examples 1 to 3 and Comparative Examples 1 to 6, mouse-derived preadipocyte cell line 3T3-L1 cells were used to inhibit adipocyte differentiation and lipid accumulation in adipocytes.

In order to evaluate the effect of inhibiting differentiation in the process of inducing differentiation from 3T3-L1 cells, a pre-adipocyte cell line, to adipocytes and the effect of inhibiting lipid accumulation after induction of differentiation, cells were plated in a 24-well plate at the concentration of 5×10⁴ cells/well using DMEM (Dulbecco's Modified Eagle Medium) medium, and cultured in the plate under the condition of 37°C and 5% CO₂ until they reached 100% confluency. When the 3T3-L1 cells reached 100% confluency, the medium was replaced with fresh DMEM medium and further cultured for 2 days. After 2 days, DMEM differentiation induction medium containing 10% FBS, 1 µM dexamethasone (Dex), 0.5 mM 3-isobutyl-1-methylxanthine (IBMX), and 2 µ g/mL insulin was added to the wells to induce differentiation into adipocytes, and then the cells were cultured for 2 days under the condition of 37°C and 5% CO₂.

After 2 days, each well was treated with DMEM medium containing 10% FBS and 2 µg/mL insulin and cultured for 2 days. After 2 days, DMEM medium containing 10% FBS was treated in each well, and the medium was exchanged every 2 days and further cultured for 6 days. From the time of treatment of the differentiation induction medium, Examples 1 to 3 and Comparative Examples 1 to 6 were mixed with the medium and treated by concentration whenever the medium was exchanged.

After adipocyte differentiation and intracellular lipid accumulation were completed, each well was treated with 10% formalin solution for 1 hour and the cells were fixed at room temperature. After cell fixation, washing with 60% isopropanol and oil red o staining were performed. Adipocyte differentiation was evaluated through a microscope after oil red o staining, and after differentiation was confirmed, lipid globules stained with 100% isopropanol were eluted and absorbance was measured at 520 nm using a micro-plate reader. At this time, the group without the sample was used as a control, and the lipid accumulation inhibition activity (%) of each sample in adipocytes was calculated as in Equation 2 below. Lipid accumulation inhibitory activity in adipocytes (%) = [(1 - (absorbance of sample treated group / absorbance of untreated group)) × 100]

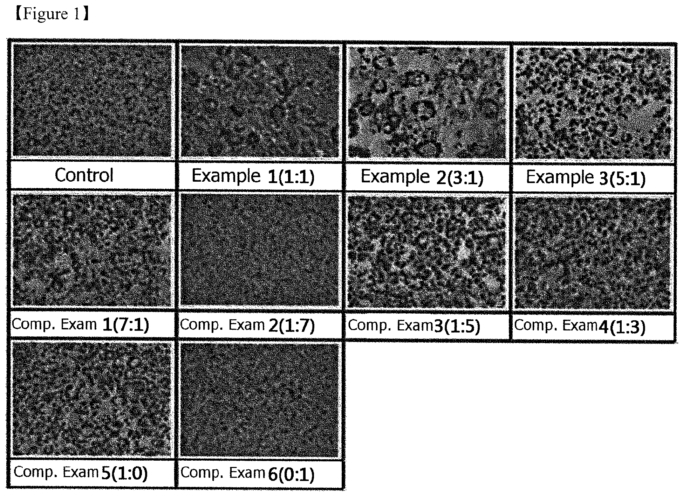

As a result, as can be seen in Figures 1 to 2, it was found that Examples 1 to 3 were more effective in inhibiting differentiation of adipocytes and inhibiting lipid accumulation in adipocytes than Comparative Examples 1 to 6. In addition, in the complex of Ashwagandha extract and Siberian chrysanthemum extract, the synergistic effect on the inhibition of differentiation of adipocytes and the inhibition of lipid accumulation in adipocytes was excellent when the complex ratio is 1: 1 to 5: 1 (Asiaganda extract: Siberian chrysanthemum extract), and when the complex ratio was out of the above range, it was found that the anti-obesity effect by inhibiting lipid accumulation was significantly reduced.

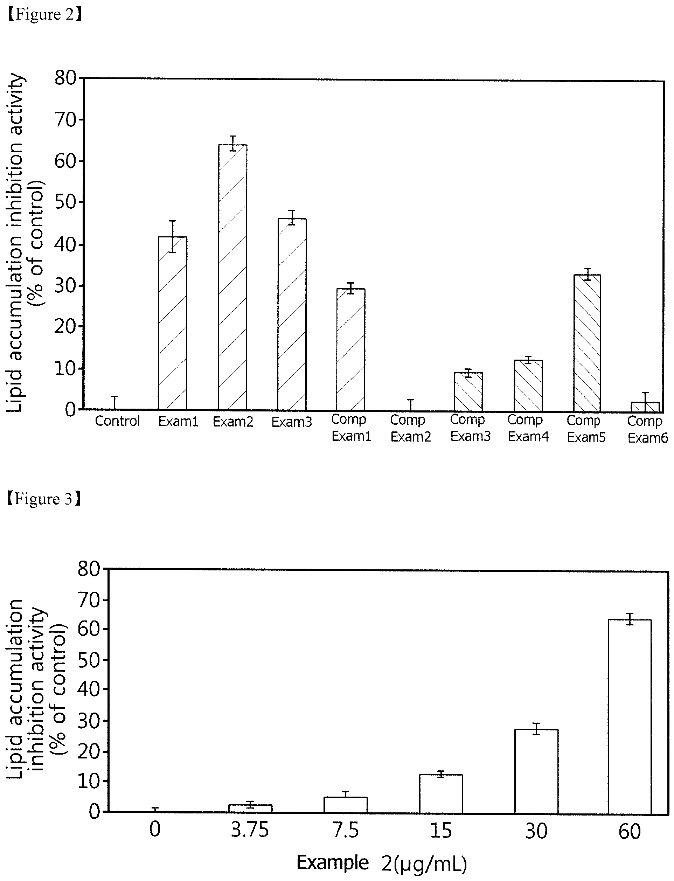

In addition, as shown in Figure 3, when the complex of Ashwagandha extract and Siberian chrysanthemum extract of Example 2 was treated for each concentration, a concentration-dependent increase in the inhibition of lipid accumulation in adipocytes was observed, thereby confirming anti-obesity effect by inhibition of fat accumulation.

### <Experimental Example 4> Inhibition of lipid digestion and absorption: Pancreatic lipase inhibitory activity

In order to determine the lipid absorption inhibitory effect using Examples 1 to 3 and Comparative Examples 1 to 6, pancreatic lipase inhibitory activity was evaluated.

Pancreatic lipolytic enzyme is an enzyme that hydrolyzes triglycerides into monoglycerides and fatty acids, and helps to digest and absorb lipids in the intestine. Thus an anti-obesity effect can be obtained by inhibiting the activity of pancreatic lipolytic enzyme and thereby making lipids excreted outside the body without being digested or absorbed in the body.

Each of Examples 1 to 3 and Comparative Examples 1 to 6 was dissolved to a final concentration of 100 µg/mL in an enzyme buffer (10 mM MOPS, 1 mM EDTA, pH 6.8) with 2.5 mg/mL of porcine pancreatic lipase, mixed with Tris-HCL buffer (pH 7.0), and reacted at 37°C for 15 minutes. Then, it was mixed with a substrate of 10 mM concentration of p-nitrophenyl butyrate (p-NPB) dissolved in N,N-Dimethylformamide (DMF), reacted at 37 °C for 30 minutes, and then put it in a 96-well plate and absorbance was measured at 405 nm using a micro-plate reader. At this time, the group without the sample was used as a control, and the pancreatic lipase inhibition activity (%) of each sample was calculated as shown in Equation 3 below. Pancreatic lipolysis inhibitory activity (%) = [{ 1 - ((absorbance of sample treated group - blank absorbance of sample treated group) / (absorbance of control group - blank absorbance of control group))} × 100]

**[Table 6]**

| Sample (complex ratio) | Treatment concentration | Pancreatic Lipase Inhibitory Activity (%) |
|---|---|---|
| Example 1(1:1) | 100 *µ*g/mL | 21.92±1.52 |
| Example 2(3:1) | 100 *µ*g/mL | 28.07±2.81 |
| Example 3(5:1) | 100 *µ*g/mL | 24.32±3.12 |
| Comparative Example 1 (7:1) | 100 *µ*g/mL | 11.47±2.63 |
| Comparative Example 2 (1:7) | 100 *µ*g/mL | 7.02±2.01 |
| Comparative Example 3 (1:5) | 100 *µ*g/mL | 6.14±1.04 |
| Comparative Example 4 (1:3) | 100 *µ*g/mL | 5.26±2.35 |
| Comparative Example 5 (1:0) | 100 *µ*g/mL | 10.8±2.41 |
| Comparative Example 6 (0:1) | 100 *µ*g/mL | 8.78±1.70 |

As a result, as shown in Table 6, it was found that when each 100 µg/mL of Examples 1 to 3 was treated, the pancreatic lipolytic enzyme inhibitory activity was 21.92% to 28.07%, which was remarkably superior compared to the pancreatic lipolytic enzyme inhibitory activity of 5.26% to 11.47% when each 100 µg/mL of Comparative Examples 1 to 6 was treated. Among them, the pancreatic lipolytic enzyme inhibitory activity of Example 2 was found to be the most excellent, and it was confirmed that there was an anti-obesity effect by inhibiting lipid absorption.

Having described specific parts of the present invention in detail above, it will be clear to those skilled in the art that these specific descriptions are only preferred embodiments, and the scope of the present invention will not limited thereby. Accordingly, the substantial scope of the present invention will be defined by the appended claims and their equivalents.

## Claims

1. A pharmaceutical composition for preventing or treating obesity, comprising a complex of an extract of Ashwagandha (*Withania somnifera*) and an extract of Siberian chrysanthemum (*Chrysanthemum zawadskii Herbich var. latilobum (Maxim.) Kitamura*) as an active ingredient.

2. The pharmaceutical composition according to claim 1, wherein the extract of Ashwagandha and the extract of Siberian chrysanthemum are extracted with a solvent selected from the group consisting of water, lower alcohols having 1 to 4 carbon atoms, and mixed solvents thereof.

3. The pharmaceutical composition according to claim 1, wherein the extract of Ashwagandha and the extract of Siberian chrysanthemum are extracted at 40 to 100 °C.

4. The pharmaceutical composition according to claim 1, wherein the complex of the extract of Ashwagandha and the extract of Siberian chrysanthemum is a dry powder.

5. The pharmaceutical composition according to claim 1, wherein the extract of Ashwagandha and the extract of Siberian chrysanthemum are mixed in the complex in a weight ratio of 1: 1 to 5: 1 (extract of Ashwagandha : extract of Siberian chrysanthemum).

6. The pharmaceutical composition according to claim 1, wherein the complex of the extract of Ashwagandha and the extract of Siberian chrysanthemum has an inhibitory effect on differentiation of adipocytes, an inhibitory effect on lipid accumulation in adipocytes, and an inhibitory activity on pancreatic lipase.

7. A pharmaceutical product comprising the composition according to any of claims 1 to 6.

8. A food composition for preventing or improving obesity, comprising a complex of an extract of Ashwagandha (*Withania somnifera*) and an extract of Siberian chrysanthemum (*Chrysanthemum zawadskii Herbich var. latilobum (Maxim.) Kitamura*) as an active ingredient.

9. The food composition according to claim 8, wherein the extract of Ashwagandha and the extract of Siberian chrysanthemum are extracted with a solvent selected from the group consisting of water, lower alcohols having 1 to 4 carbon atoms, and mixed solvents thereof.

10. The food composition according to claim 8, wherein the extract of Ashwagandha and the extract of Siberian chrysanthemum are extracted at 40 to 100□.

11. The food composition according to claim 8, wherein the complex of the extract of Ashwagandha and the extract of Siberian chrysanthemum is a dry powder.

12. The food composition according to claim 8, wherein the extract of Ashwagandha and the extract of Siberian chrysanthemum are mixed in the complex in a weight ratio of 1: 1 to 5: 1 (extract of Ashwagandha : extract of Siberian chrysanthemum).

13. The food composition according to claim 8, wherein the complex of the extract of Ashwagandha and the extract of Siberian chrysanthemum has an inhibitory effect on differentiation of adipocytes, an inhibitory effect on lipid accumulation in adipocytes, and an inhibitory activity on pancreatic lipase.

14. A health functional food product comprising the composition according to any of claims 8 to 13.

15. A food product comprising the composition according to any of claims 8 to 13.
